(19) Europäisches Patentamt
European Patent Office
Office européen des brevets

(11) Publication number: **0 065 513**
**B1**

# (12) EUROPEAN PATENT SPECIFICATION

(45) Date of publication of patent specification: **18.09.85**

(51) Int. Cl.[4]: **A 61 K 31/71,** A 61 K 31/12

(21) Application number: **80902224.7**

(22) Date of filing: **27.11.80**

(86) International application number:
**PCT/HU80/00005**

(87) International publication number:
**WO 82/01821 10.06.82 Gazette 82/15**

## (54) ANTIMYCOTIC SYNERGISTIC PHARMACEUTICAL COMPOSITIONS AND PROCESS FOR THE PREPARATION THEREOF.

(43) Date of publication of application:
**01.12.82 Bulletin 82/48**

(45) Publication of the grant of the patent:
**18.09.85 Bulletin 85/38**

(84) Designated Contracting States:
**DE FR GB**

(56) References cited:
**FR-M- 6 072**

**CHEMICAL ABSTRACTS, vol. 95, no. 24, December 14, 1981, page 377, abstract no. 209647x COLUMBUS OHIO (US)**
**CHEMICAL ABSTRACTS, vol. 84, no. 3, January 19, 1976, page 15, column 1, abstract no. 12316e COLUMBUS OHIO (US)**
**M.D. Mashkovskiy "Medicines", part II, published 1977, publishing house Medicine (Moscou), see page 209**
**I.S. Azhgikhine "Techniques of Preparing Medicines", published 1975, publishing house Medicine (Moscou), see pages 297, 335, 385, 406**

(73) Proprietor: **CHINOIN Gyogyszer és Vegyészeti Termékek Gyára RT.**
**To utca 1-5**
**H-1045 Budapest IV (HU)**

(72) Inventor: **KOVACS, András**
**19, Bajcsy-Zs. ut**
**H-1215 Budapest (HU)**
Inventor: **NEMES, József**
**9, Csaktornya u**
**H-1142 Budapest XIV (HU)**
Inventor: **SZEBENI, Rudolf**
**11, Hébelt Ede u.**
**H-1026 Budapest (HU)**
Inventor: **NAGYKALDI, Attila**
**23, Dorozsmai ut.**
**H-1142 Budapest (HU)**

(74) Representative: **Skailes, Humphrey John et al**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 065 513 B1

(56) References cited:

**Referativny zhurnal "Farmakologia. Khimioterapevticheskie sredstva," N2, published in February 1977 (Moscow, VINITI). Elsner Zofia et al, "Gel with nystatin for treatment of lung mycosis", see page 121, abstract N254742. Pol. J. Pharmacol. and Pharm." 1976, 28, N4, 349-352 (Engl.)**

**Description**

The invention relates to antimycotic synergistic pharmaceutical compositions and a process for the preparation thereof.

It is known that nystatin is an antibiotic having antimycotic effect and produced by the microorganism strain Streptomyces noursei. It is also known that compositions containing nystatin and 8-hydroxyquinoline possess strong antimycotic activity (British Patent No. 1.143.998). It is also known that some 1,4-naphthoquinone derivatives exhibit certain antimycotic activity [Br. J. Pharmac. *40*, 871—770 (1970)].

It is the object of the present invention to provide antimycotic compositions which surpass on the one hand the activity of the known compositions and are active on the other against resistant strains as well.

The present invention relates to an antimycotic synergistic pharmaceutical composition comprising nystatin and a 2-lower alkyl-1,4-naphthoquinone or an addition product thereof in admixture with suitable inert pharmaceutical carriers.

The present invention also relates to a process for the preparation of an antimycotic synergistic pharmaceutical composition which comprises admixing nystatin and a 2-lower alkyl-1,4-naphthoquinone or an addition product thereof with suitable inert pharmaceutical carriers.

The present invention is based on the recognition that there is a synergistic interaction between nystatin and 2-lower alkyl-1,4-naphthoquinones with the result that in the composition of the present invention the active ingredients exert their desired antimycotic effect in a smaller concentration than when used per se.

The term "lower alkyl" used throughout the specification relates to straight or branched chain alkyl groups having 1—4 carbon atoms (e.g. methyl, ethyl, n-propyl, isopropyl, n-butyl, isobutyl).

As 2-lower alkyl-1,4-naphthoquinone component preferably the 2-methyl-1,4-naphthoquinone or 2-ethyl-1,4-naphthoquinone, particularly the 2-methyl-1,4-naphthoquinone can be used.

The addition product of the 2-lower alkyl-1,4-naphthoquinone may be any suitable and conventional adduct formed by carbonyl compounds, particularly a sodium bisulfite adduct.

The compositions of the present invention contain preferably from 20.000 to 100.000 IU (international units) of nystatin on 1 mg of 2-methyl-1,4-naphthoquinone or an addition product thereof.

According to a preferred embodiment of the present invention there are provided antimycotic synergistic compositions comprising as active ingredient 1 mg of 2-methyl-1,4-naphthoquinone sodium bisulfite adduct and 20.000—100.000 IU of nystatine.

The compositions of the present invention are preferably in forms suitable for topical (local) use particularly in the form of suppositories, aerosol, jelly and vaginal tablets; the vaginal tablets proved to be particularly advantageous.

The compositions of the present invention are prepared by methods of pharmaceutical industry known per se by using conventional solid or liquid carriers.

Suppositories are prepared by using usual suppository bases which are soluble in body fluid and melt at body temperature. As bases soluble in body fluids Massa polyoxaethani is particularly preferred. The composition can be prepared by the moulding (casting) method. The hydrophilic suppository base is melted and in a small portion thereof, the active ingredients are suspended and the suspension is cast into forms in a suitable apparatus equipped with a pretreated stirrer. The suppositories may be of the conventional torpedo-shaped form or flattened ovula form.

Suppository bases melting at body temperature may also be used, such as Adeps solidus (Witepsol 35) or cocoa butter). The suppositories can be prepared by the casting or pressing procedures by methods known per se.

The aerosol compositions can also be prepared by methods known per se. With the aid of non-ionic surfactants an oil-in-water type emulsion is prepared, the inner fatty phase thereof being 15—25%. As fatty phase Ung. emulsificans non-ionicum (see VI. Hungarian Pharmacopoeia) can be used. The active ingredients are dispersed in the emulsion. The system thus obtained, which is free of any sedimentations due to the viscosity thereof, is filled into an aerosol flask and set under the pressure of a suitable propellant. For this purpose conventional propellants can be used e.g. the halogenated hydrocarbons (Freon®12 or Freon 114).

The vaginal tablets contain conventional carriers (e.g. starch, lactose, auxiliary agents). The tablets can be prepared by dry or wet granulating methods. In order to obtain suitable homogeneity it is preferred to use the wet method. It is expedient to add disintegrating agents and auxiliary agents (e.g. talc, magnesium stearate, Aerosil®) to the granules prior to forming the tablets. As disintegrating agents preferably Polyplasdone XL, Esmospreng and Primojel can be used.

The active ingredients can also be formed as semi-solid compositions (e.g. ointment). As carriers preferably mineral oil fractions, hydrogenated or non-hydrogenated triglycerides and oils of animal or vegetable origin and jellies of hydrophobic polymers of synthetic or semi-synthetic origin can be used. The active ingredients are distributed in the carrier by suspending the same. The ointments can be preferably filled in tubes having an elongated discharge opening.

According to a preferred embodiment of our invention there are provided pharmaceutical compositions comprising pro 1 g composition 100.000 IU of nystatin and 1.85 mg of 2-methyl-1,4-naphthoquinone or an equivalent amount of another 2-lower alkyl-1,4-naphthoquinone or an addition product thereof.

The compositions of the present invention are suitable for the treatment of leukorrhoea (fluor) and infections caused by Candida albicans and Trichomonas vaginalis. Until the twentieth week of pregnancy the compositions are active in the treatment of leukorrhoea (fluor), trichomoniasis and oidiomycosis (thrush, soor) and when administered to patients taking oral anti-conceptive pills the compositions of the present invention stop chronic leukorrhoea (fluor) and eliminate the susceptibility to infections.

The synergistic activity of the compositions of the present invention is shown by the following tests.

In vitro test

Candida albicans and Trichomonas vaginalis strains are cultured in a liquid nutrient medium. Incubation is carried out at 37°C for 48 hours. Each nutrient medium (10 ml) is inoculated with 1000 germs per ml. The number of germs is determined in a Bürker chamber. Dilution series are prepared from the stock inoculum by using a physiological sodium chloride solution. To the mixture of the nutrient medium and 0.5 ml a physiological sodium chloride solution 2-methyl-1,4-naphthoquinone, nystatin and mixtures thereof are added.

It has been found that 2-methyl-1,4-naphthoquinone inhibits the growth of Candida albicans and Trichomonas vaginalis in a minimal inhibitory concentration of 5 mg/10 ml. When used in a smaller concentration it is completely inactive.

The minimal inhibitory concentration of nystatin is determined in the manner disclosed above.

1.25 mg/10 ml of 2-methyl-1,4-naphthoquinone is added to an amount of nystatin being the one fiftieth (1/50) part of the minimal inhibitory concentration. Complete inhibition of the growth of Candida albicans and Trichomonas vaginalis was observed.

This means that 2-methyl-1,4-naphthoquinone, when used at a concentration completely inactive per se, strongly potentiates the effect of nystatin.

In vivo test

Patients suffering of vulvovaginitis candidomycetica are treated with suppositories according to Example 1. As control suppositories containing no 2-methyl-1,4-naphthoquinone-sodium sulfite adduct but nystatin only are used. The results are summarised in the following Table. Number of patients 35.

| Test compound | Recovery, in % of the cases |
|---|---|
| 2-Methyl-1,4-napthoquinone-sodium bisulfite adduct+nystatin | 88 |
| Nystatin | 66 |

Industrial application

The compositions according to the present invention can be used in therapy for the treatment of infections caused by Candida albicans and Trichomonas vaginalis. In clinical tests they proved to show a high synergistic effect in the treatment of vulvovaginitis candidomycetica. The duration of the treatment depends on various factors e.g. the type of infection and the condition of the patient. It can vary over wide ranges and is preferably 10—12 days.

Further details and the best modes for carrying out the invention are shown in the following Examples without limiting the scope of protection to the said Examples.

Example 1

Suppositories of the following composition are prepared:

| Component | Amount |
|---|---|
| Sodium bisulfite adduct of 2-methyl-1,4-naphthoquinone | 5 mg |
| Nystatin | 100.000 IU |
| Vehiculum | 2.75 g |

The vehiculum (Massa polyoxyethani) is melted on a water bath at 45—50°C. In a small portion of this vehiculum first the sodium bisulfite adduct of 2-methyl-1,4-naphthoquinone and thereafter the nystatin are suspended and the mixture is quickly poured into moulds at the above temperature in a suitable

conventional apparatus. The moulds are cooled with water. The suppositories are removed from the moulds and packed under the exclusion of air.

Example 2

Suppositories of the following composition are prepared:

| Component | Amount |
|---|---|
| Sodium bisulfite adduct of 2-methyl-1,4-naphthoquinone | 5 mg |
| Nystatin | 250.000 IU |
| Vehiculum | 2.7 g |

The vehiculum (cocoa butter) is melted on a water bath at 40°C and the active ingredients are suspended in a small amount thereof. The suppositories are formulated in an analogous manner to the process described in Example 1.

As vehiculum in the place of cocoa butter Adeps solidus (Witepsol 35) can be used too which melts at a temperature between 40°C and 45°C.

Example 3

5 mg of 2-methyl-1,4-naphthoquinone sodium bisulfite adduct and 200.000 IU of nystatin are continuously admixed with small amounts of cocoa butter. The homogenised mixture is pressed into suppositories with the aid of an Eagler machine.

Example 4

An emulsion is prepared from the following components:

| Component | per 100 g emulsion |
|---|---|
| Ung. emulsificans non-ionicum | 20 g |
| Distilled water | ad 100 g |

The composition of the Ung. emulsificans non-ionicum is as follows:

| | |
|---|---|
| Sorboxaethanum stearinicum | 10% |
| Liquid paraffin | 10% |
| Cetyl stearyl alcohol | 30% |
| Vaseline® album | 50% |

The non-ionic emulsifier is prepared by admixing the components on a water bath and stirring the mixture until it gets cold:

20 g of a suppository base meeting the requirements of pharmacopoeias and 50 g of water are converted on a water bath into an oil-water type emulsion and cooled to 20°C. The active ingredients (92,5 mg of the sodium bisulfite adduct of 2-methyl-1,4-naphthoquinone and 5.000.000 IU of nystatin) are suspended in 20 g of water and the suspension is admixed with the emulsion prepared above. No components precipitate from the viscous system.

The mixture thus obtained is filled into an aerosol flask under atmospheric pressure and then set under Freon® pressure. The propellant used is of the following composition:

| | |
|---|---|
| Freon®12 | 40% |
| Freon®114 | 60% |

The composition leaves the flask as a foam.

Example 5

Vaginal tablets having the following composition are prepared:

| Component | Amount |
|---|---|
| Sodium bisulfite adduct of 2-methyl-1,4-naphthoquinone | 185 mg |
| Nystatin | 10.000.000 IU |
| Lactose | 60 g |
| Potato starch | 30 g |
| Polyvidonum | 5 g |
| Polyplasdon-XL | 1.5 g |
| Talc | 3 g |
| Magnesium stearate | 2 g |
| Aerosil® | 1 g |

A mixture of the lactose and potato starch is granulated with the aid of a sufficient amount of polyvinyl pyrrolidone dissolved in 96% ethanol; in the said alcoholic solution the active ingredients were previously suspended. The granules are dried at a temperature below 25°C (in a fluidisation drier) and the homogenised and sieved mixture of the other additives (Polyplasdon-XL, talc, magnesium stearate and aerosil) is added thereto. The mixture thus obtained is pressed into tablets weighing 2 g.

The trade marks and commercial names used throughout the specification relate to the following products:

Adeps solidus=a mixture of mono-, di- and triglycerides of saturated carboxylic acids (from $C_{11}H_{23}COOH$ to $C_{17}H_{35}COOH$); (Ph.Hg.VI.)

Cocoa butter=this is a mixture of the fatty triglycerides pressed from the roasted and peeled cocoa grains. (Ph.Hg.VI.)

Polyplasdone XL=polyvinyl pyrrolidone

Esmospreng=formaldehyde-casein

Primojel=carboxymethyl starch

Massa polyoxyethani=polyoxaethanum stearinicum 90%
polyoxaeth.—4000 5%
polyoxaeth.—1540 5%

Polyoxaeth. stear.=polyoxethane stearate

$$HO—CH_2—(CH_2—OCH_2)_n—CH_2—COO—(CH_2)_{16}—CH_3,$$

n is an integer between 2 and 40 (average), (average molecular weight: 2076)

Polyoxaethanum—4000-polyoxyethylene—4000=

$$HO—CH_2—(CH_2—O—CH_2)_n—CH_2OH$$

n=about 90 (average) (molecular weight 3600—4400)

Polyoxaeth. 1540=polyoxyethylene-1540

$$HO—CH_2—(CH_2—O—CH_2)_n—CH_2OH$$

n=about 34 (average) (molecular weight 1400—1600).

Aerosil®=acidus silicicum colloidale (colloidal silica)

Polyvidonum=polyvinyl pyrrolidone

Amylum solani=potato starch.

The additives and carriers used in the Examples meet the requirements of the VI. Hungarian Pharmacopoeia (Ph.Hg.VI.).

**Claims**

1. A pharmaceutical composition comprising nystatin and a 2-($C_{1-4}$) alkyl-1,4-naphthoquinone or an addition product thereof in admixture with one or more suitable inert pharmaceutical carriers, and

containing 20,000 to 100,000 international units (IU) of nystatin per mg of 2-methyl-1,4-naphthoquinone or an equivalent amount of another 2-($C_{1-4}$) alkyl-1,4-naphthoquinone or an addition product thereof.

2. A composition according to claim 1 which comprises 2-methyl-1,4-naphthoquinone as the 2-alkyl-1,4-naphthoquinone.

3. A composition according to claim 1 which comprises a sodium bisulfite adduct as addition product.

4. A composition according to any of claims 1 to 3 in a form suitable for topical (local) treatment, preferably suppositories, aerosol, jelly or vaginal tablets.

5. A process for the preparation of a composition according to any preceding claim which comprises admixing the active ingredients with one or more suitable inert or liquid pharmaceutical carriers.

**Revendications**

1. Une composition pharmaceutique comprenant de la nystatine et une 2-alkyl ($C_{1-4}$)-1,4-naphtoquinone ou un de ses produits d'addition en mélange avec un ou plusieurs supports pharmaceutiques inertes appropriés et contenant 20 000 à 100 000 unités internationales (UI) de nystatine par mg de 2-méthyl-1,4-naphtoquinone ou d'une quantité équivalente d'une autre 2-alkyl($C_{1-4}$)-1,4-naphtoquinone ou d'un produit d'addition correspondant.

2. Une composition selon la revendication 1 qui comprend la 2-méthyl-1,4-naphtoquinone comme 2-alkyl-1,4-naphtoquinone.

3. Une composition selon la revendication 1 qui comprend un produit d'addition du bisulfite de sodium comme produit d'addition.

4. Une composition selon l'une quelconque des revendications 1 à 3 sous une forme appropriée au traitement local, de préférence sous forme de suppositoires, d'aérosol, de gelée ou de comprimés gynécologiques.

5. Un procédé pour la préparation d'une composition selon l'une quelconque des revendications précédentes qui comprend le mélange des ingrédients actifs avec un ou plusieurs supports pharmaceutiques appropriés, inertes ou liquides.

**Patentansprüche**

1. Arzneimittelzusammensetzung, dadurch gekennzeichnet, dass sie Nystatin und ein 2-($C_{1-4}$-Alkyl)-1,4-naphthochinon oder eines seiner Additionsprodukte in Mischung mit einem oder mehreren geeigneten inerten pharmazeutischen Trägermaterialien und in einer Menge von 20 000 bis 100 000 Internationalen Einheiten (IE) Nystatin pro mg 2-Methyl-1,4-naphthochinon oder einer äquivalenten Menge eines anderen 2-($C_{1-4}$-Alkyl)-1,4-naphthochinons oder eines seiner Additionsprodukte enthält.

2. Arzneimittelzusammensetzung nach Anspruch 1, dadurch gekennzeichnet, dass sie als 2-Alkyl-1,4-naphthochinon 2-Methyl-1,4-naphthochinon enthält.

3. Arzneimittelzusammensetzung nach Anspruch 1, dadurch gekennzeichnet, dass sie als Additionsprodukt ein Natriumbisulfitaddukt enthält.

4. Arzneimittelzusammensetzung nach Anspruch 1, 2 oder 3, dadurch gekennzeichnet, dass sie in einer für die topische (lokale) Behandlung geeigneten Form, vorzugsweise als Suppositorien, Aerosol, Gelee oder Vaginaltabletten, vorliegt.

5. Verfahren zur Herstellung einer Arzneimittelzusammensetzung nach Ansprüchen 1, 2, 3 oder 4, dadurch gekennzeichnet, dass man die Wirkstoffe mit einem oder mehreren der geeigneten inerten oder flüssigen pharmazeutischen Trägermaterialien vermischt.